# EUROPEAN PATENT APPLICATION

(11) **EP 4 000 533 A1**
(43) Date of publication of application: **25.05.2022**
(21) Application number: 20209627.7
(22) Date of filing: 24.11.2020
(51) Int. Cl.: A61B 10/00

(54) **DEVICE AND KIT FOR COLLECTING AND RELEASING A LIQUID SAMPLE**

(30) Priority: 19.11.2020 PT 2020116892
(71) Applicant: Genotrix, S.A., 4785-107 Trofa (PT)
(72) Inventor: Albert Amar, Frederic, 1205 Geneve (CH)
(74) Representative: Patentree

(57) **Abstract**

The present solution describes a fluid sample collector, production methods and uses thereof, namely a device for collecting biological fluids, body fluids, mineral and organic liquids, food samples, beverage samples, among others. Device for collecting and releasing a liquid sample comprising: an incompressible porous elongated container for collecting and releasing the liquid sample; a holder connected to an end of the container; wherein said container comprises a plurality of sub-compartments wherein said sub-compartments are divided by perforated partitions. Kit comprising the device and a receptacle, in particular the receptacle being a test tube or a vial, further in particular a cryovial.

## Description

### TECHNICAL FIELD

The present solution describes a fluid sample collector, production methods and uses thereof, namely a device for collecting biological fluids, body fluids, mineral and organic liquids, food samples, beverage samples, among others.

### BACKGROUND

A problem present in the state of the art is the urgent need to find more alternatives to collect saliva for SARS CoV-2 detection since current manufacturers around the world don't have enough production capacity and the current existing solutions aren't safe and properly adapted to the current pandemic situation.

An existing solution to this problem is the devices for saliva collection where the patient must spit into it or, in alternative, small cotton sticks that the patient must chew and then put in a safe and proper container.

Document US20150133817 relates to a device for obtaining saliva samples from infants, including premature infants. The device includes a pacifier portion, a stiffener, a collector, and a pad compression tube.

Document WO2008139324 relates to devices, kits for fluid sample collection, and methods of using them. The subject devices and kits are particularly useful for collection of biological samples, particularly bodily fluids. The devices comprise a collection member comprising a first absorbent material in fluid communication with an indicator assembly comprising a second absorbent material.

Document EP1541986 relates to specimen sampling device for investigating specimen liquid, has manually operated pumping device with which specimen liquid and dilution liquid can be dispensed simultaneously or successively The specimen sampling device has a sampling element for specimen liquid and a preferably integrated reservoir for a dilution liquid and is portable.

Document US20040022687 relates to a device for collecting and releasing a sample liquid, especially saliva as well as a system and method are provided. The device obtains and provides a defined saliva sample, i.e., a filtered and homogeneously mixed saliva sample of a predetermined volume, in the simplest possible manner. The device has a sampling tip made of an incompressible and porous material, which is moved with its mouthpiece in the mouth of a test subject for 1 to 2 minutes and collects saliva based on the capillary action.

However, these existing solutions create a lot of difficulties and risks of contamination during the process. When the patient is spitting into a cup or device, we are increasing the chances of contaminating the health professional in charge of the process. It is also a non-hygienic procedure as saliva may pour outside the containers and, again, put health professionals in danger. These solutions also require that saliva is then transferred to the final tube for analysis increasing the needs of manipulation on the samples.

These facts are disclosed in order to illustrate the technical problem addressed by the present disclosure.

### GENERAL DESCRIPTION

The present solution describes a fluid sample collector, production methods and uses thereof, namely a device for collecting biological fluids, body fluids, mineral and organic liquids, food samples, beverage samples, among others.

The fluid collector device of the present disclosure comprises the creation of a liquid retention chamber, without the use of any fibres, with perforated internal sub-compartments to maximize the retention of the collected fluids and advantages thereof include are ease of use, great comfort for patient (when collecting body fluids) great fluid retention capacity, absence of fibres or other materials that can interfere with the analysis processes of collected fluids.

The present disclosure describes aa fluid sample collector comprising a overall cylindrical shape device produced through additive manufacturing which includes a cylindrical perforated tip with capillary holes to allow fluid passage to an internal reservoir segmented in sub-compartments divided by perforated disks. The tip has blades in the top and bottom of the surface to ensure that, if required, the collected fluids can be properly mixed with other fluids.

The solution of the present disclosure uses a solid chamber for collection, with no fibres or absorbent material being necessary. The sub-compartments help to grab more fluids and assure its retention inside the chamber leading to higher volumes collected. There is no use of internal or external capillary fibres or tubes. The device is preferably designed to fit inside a matching tube, small cryovials or any diagnostic tube above, for example, 10 mm of diameter. It preferably has a breaking point to allow tubes to be closed with a cap and keep the device inside.

The advantages of the present disclosure in respect of the prior art include a safer procedure for the health professional in charge of the patient, a cleaner process for patient and health professional. The usage of high quality resins (biocompatible when required) avoid any cross-contamination from fibres or other materials during the collection and analysis process. The ergonomic shape allows great conform for patients (when collecting body fluids). By being possible to use it directly in a sampling tube, we avoid additional manipulation, reducing the risks of cross-contamination or sample damage.

It is disclosed a device for collecting and releasing a liquid sample comprising:
an incompressible porous elongated container for collecting and releasing the liquid sample;
a holder connected to an end of the container;
wherein said container comprises a plurality of sub-compartments wherein said sub-compartments are divided by perforated partitions.

In an embodiment, the container has external pores for allowing the liquid sample to enter and exit the container.

In an embodiment, the partitions have capillary pores for allowing the liquid sample to move between sub-compartments.

In an embodiment, the sub-compartments are sufficiently broad such as not to be capillary.

In an embodiment, the device is obtained by additive manufacturing of layer upon layer from 3D model data, in particular 3D-printing. This is very useful for obtaining a fully enclosed (other than for the disclosed perforations) container which otherwise would not be possible to obtain by classical mould injection. In particular, the device is post-cured after 3D-printing, for example by thermal or UV treatment.

In an embodiment, an end of the container opposite the holder end comprises a plurality of end perforations.

In an embodiment, the end perforations are capillary.

In an embodiment, the holder is breakable for separating away from the container, in particular the holder comprising a weakened portion which is breakable for separating away from the container.

In an embodiment, an end of the container opposite the holder end comprises a plurality of fins for dripping the liquid sample.

In an embodiment, the container is cylindrical.

In an embodiment, the container comprises a cylindrical part and a cone-shaped part with a rounded end.

In an embodiment, the partitions are circular.

In an embodiment, the holder comprises a shaft for holding the device.

In an embodiment, the shaft comprises longitudinal fins extending along a part of the shaft.

In an embodiment, the pores have a diameter of 0.5-1.5 mm, preferably 0.7-1.3 mm, more preferably 1-1.3 mm.

In an embodiment, the container has a length of 5-35 mm, in particular 10-30 mm, further in particular 15-25 mm.

In an embodiment, the container has a diameter of 3-14 mm, in particular 5-12 mm, further in particular 8-10 mm.

In an embodiment for biological fluid collection, the container has a diameter of 3mm to 14mm and a length of 5mm to 35 mmm.

In an embodiment for general fluid collection, the container has a diameter of 3mm to 25mm and a length of 5mm to 50 mmm.

In an embodiment for chemical and food fluid collection, the container has a diameter of 5mm to 25mm and a length of 5mm to 35 mm.

In an embodiment, the device material is sterilizable by autoclave or gamma-ray.

In an embodiment, the device material is a biocompatible or a non-toxic resin.

In an embodiment, the device material is selected from urethane, methacrylate, dimethacrylate, bismethacrylate, or mixtures thereof.

In an embodiment, the container has been previously pre-treated with surfactant and dried.

In an embodiment, the device is transparent or translucent.

It is also disclosed a kit comprising the device of any of the described embodiments and a receptacle, in particular the receptacle being a test tube or a vial, further in particular a cryovial.

In an embodiment, the receptacle comprises a liquid medium, in particular the liquid medium being a buffer solution, a saline solution or a physiological saline solution, a conservative medium or a freezing agent medium.

In an embodiment, the device is storable in the receptacle, in particular the receptacle having a cap for closing the receptacle with the device stored in the receptacle.

In an embodiment, an outer diameter of the container fits an inner diameter of the receptacle such that a suction force is obtained by a reciprocating motion of the container in respect of the receptacle for releasing the liquid sample from the container into the receptacle.

It is also disclosed a use of the device for liquid sampling as a swab.

It is also disclosed a use wherein said liquid is a body fluid or a foodstuff liquid.

It is also disclosed a use wherein the body fluid is saliva, vaginal liquid, or tear liquid.

It is also disclosed a use wherein the foodstuff liquid is wine, soup, juice, or jam.

It is also disclosed a use for diagnostic tests such as infectious diseases diagnostic tests, in particular for COVID-19 diagnostic tests.

### BRIEF DESCRIPTION OF THE DRAWINGS

The following figures provide preferred embodiments for illustrating the disclosure and should not be seen as limiting the scope of invention.

The figure drawings associated with this disclosure typically are not drawn with dimensional accuracy to scale, these drawings have been drafted with a focus on clarity of viewing and understanding rather than dimensional accuracy.

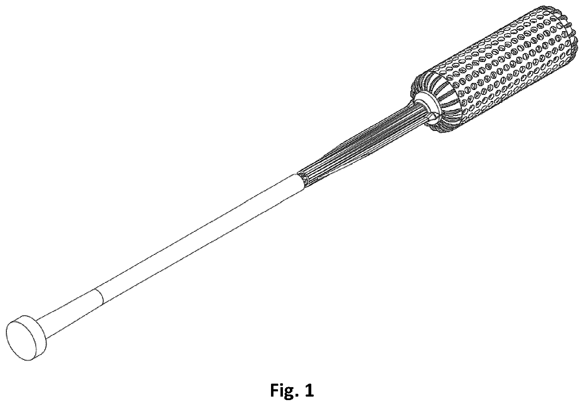

**Figure 1****:** Schematic representation of an embodiment of a swab solution for saliva collection (perspective).
**Figure 2****:** Schematic representation of an embodiment of a swab Functional Components (exploded view).
   a - represents the flat round base used to improve handling during usage,
   b - represents the main shaft used to enlarge amplitude of movement in collection phase,
   c - represents the breakpoint of the shaft used to allow breakage and storage of swab inside the tube,
   d - represents the shaft with fins to allow sample mixing when required,
   e - represents the collector Head base with fins to allow for mixing,
   f - represents the collector main body with exterior holes to allow for saliva collection,
   g - represents the internal perforated discs that allow saliva to be maintained inside,
   h - represents the collector Head top with fins to allow for mixing.

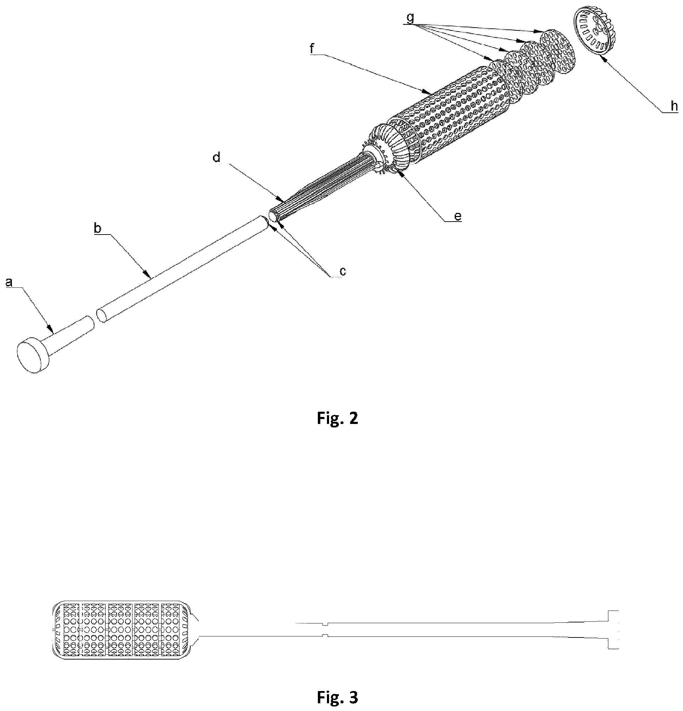

**Figure 3****:** Schematic representation of an embodiment of a swab Cross-Section front-view (internal view of collector head) .

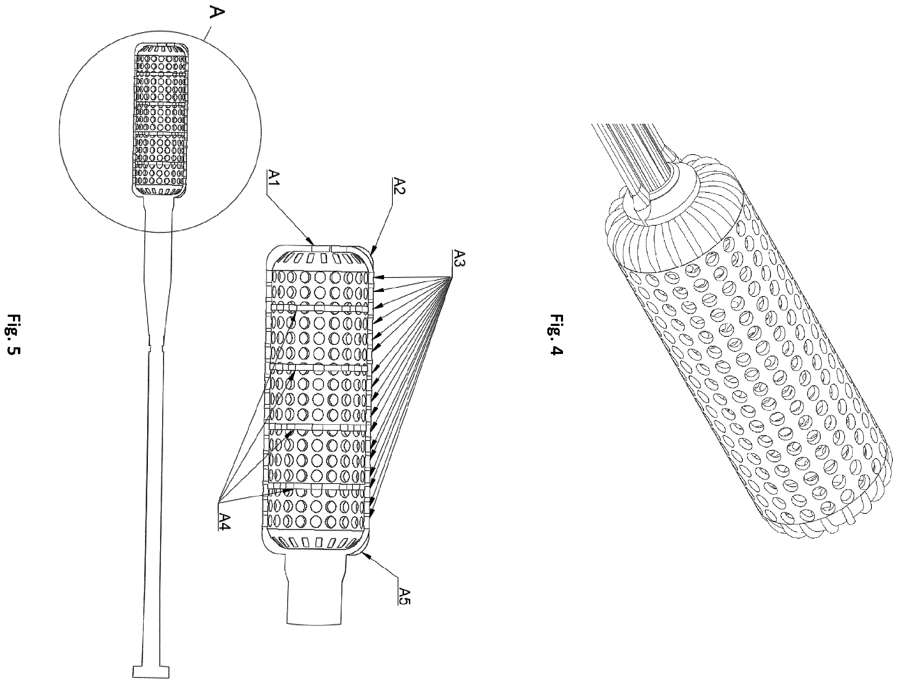

**Figure 4****:** Schematic representation of an embodiment of a detail of collector head.
**Figure 5****:** Schematic representation of an embodiment of a swab cross-section Sideview (Detail of collector head) wherein
   A1- represents the collector head top holes,
   A2- represents the collector head top fins,
   A3- represents the collector head side holes,
   A4- represents the collector head internal perforated discs,
   A5- represents the collector head bottom fins.

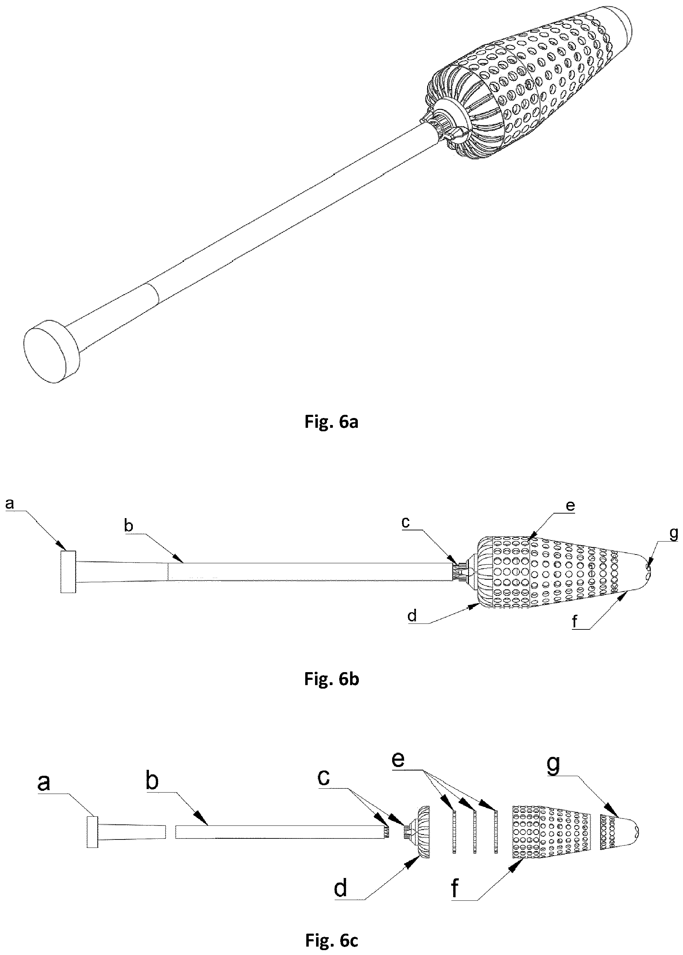

**Figure 6****:** Schematic representation of an alternative embodiment of a disclosed device for being used as a swab with a collector receptacle with a closure cap, in (a) perspective view, (b) side view and (c) exploded view, wherein
   a - represents the flat round base used to improve handling during usage,
   b - represents the main shaft used to enlarge amplitude of movement in collection phase,
   c - represents the breakpoint of the shaft used to allow breakage and storage of swab inside the tube,
   d - represents the collector Head base with fins to allow for mixing,
   e - represents internal partitions, internal perforated discs that allow saliva to be maintained inside,
   f - represents the collector main body with exterior holes to allow for saliva collection,
   g - represents the collector head top with fins to allow for mixing.

### DETAILED DESCRIPTION

The present solution describes a fluid sample collector, production methods and uses thereof, namely a device for collecting biological fluids, body fluids, mineral and organic liquids, food samples, beverage samples, among others.

The fluid collector device of the present disclosure comprises the creation of a liquid retention chamber, with perforated internal sub-compartments to maximize the retention of the collected fluids and advantages thereof include are ease of use, great comfort for patient (when collecting body fluids) great fluid retention capacity, absence of fibres or other materials that can interfere with the analysis processes of collected fluids.

Optionally, the fluid collector device may use an absorbent material inside the collection container, but this is not required as the device is capable of collecting liquids by the way it is shaped.

The term "comprising" whenever used in this document is intended to indicate the presence of stated features, integers, steps, components, but not to preclude the presence or addition of one or more other features, integers, steps, components or groups thereof. Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof. The above described embodiments are combinable. The following claims further set out particular embodiments of the disclosure.

## Claims

1. Device for collecting and releasing a liquid sample comprising:
an incompressible porous elongated container for collecting and releasing the liquid sample;
a holder connected to an end of the container;
wherein said container comprises a plurality of sub-compartments wherein said sub-compartments are divided by perforated partitions.

2. Device according to the previous claim wherein the container has external pores for allowing the liquid sample to enter and exit the container.

3. Device according to any of the previous claims wherein the partitions have capillary pores for allowing the liquid sample to move between sub-compartments.

4. Device according to any of the previous claims wherein the sub-compartments are sufficiently broad such as not to be capillary.

5. Device according to any of the previous claims wherein the device is obtained by additive manufacturing of layer upon layer from 3D model data, in particular 3D-printing.

6. Device according to any of the previous claims wherein an end of the container opposite the holder end comprises a plurality of end perforations.

7. Device according to the previous claim wherein the end perforations are capillary.

8. Device according to any of the previous claims wherein the holder is breakable for separating away from the container, in particular the holder comprising a weakened portion which is breakable for separating away from the container.

9. Device according to any of the previous claims wherein the container is cylindrical.

10. Device according to any of the claims 1-9 wherein the container comprises a cylindrical part and a cone-shaped part with a rounded end.

11. Device according to any of the claims 10-11 wherein the partitions are circular.

12. Device according to any of the previous claims wherein the container has been previously pre-treated with surfactant, in particular pre-treated with a surfactant solution and subsequently dried.

13. Device according to any of the previous claims wherein the device is transparent or translucent.

14. Kit comprising the device described in any of the previous claims and a receptacle wherein the device is storable in the receptacle, in particular the receptacle being a test tube or a vial, further in particular a cryovial.

15. Kit according to the previous claim wherein an outer diameter of the container fits an inner diameter of the receptacle such that a suction force is obtained by a reciprocating motion of the container in respect of the receptacle for releasing the liquid sample from the container into the receptacle.
